Europäisches Patentamt

European Patent Office

Office européen des brevets

(11) Publication number: **0 090 617**
**B1**

(12) **EUROPEAN PATENT SPECIFICATION**

(45) Date of publication of patent specification: **18.01.89**

(21) Application number: **83301689.2**

(22) Date of filing: **25.03.83**

(51) Int. Cl.⁴: **A 61 K 39/09,** C 12 N 15/00 //
C12R1/46

(54) Vaccine for preventing or inhibiting human dental caries induced by cariogenic Streptococcus mutans.

(30) Priority: **25.03.82 JP 47970/82**
**19.05.82 JP 47970/82**
**15.02.83 JP 23200/83**

(43) Date of publication of application:
**05.10.83 Bulletin 83/40**

(45) Publication of the grant of the patent:
**18.01.89 Bulletin 89/03**

(84) Designated Contracting States:
**AT BE CH DE FR GB IT LI LU NL SE**

(56) References cited:
**GB-A-1 375 866**
**US-A-4 133 875**

(73) Proprietor: **KITASATO KENKYUSHO**
**9-1, Shirokane 5 chome Minato-ku**
**Tokyo-to (JP)**

(72) Inventor: **Tsurumizu, Takashi**
**3-4-4, Konodai**
**Ichikawa-shi Chiba-ken (JP)**
Inventor: **Sato, Makoto**
**2-31, Otsubo-jutaku, 232-1 Otsubo, Hachiman-cho**
**Tokushima-shi, Tokushima-ken (JP)**
Inventor: **Hashimoto, Takashi**
**1-21, Kami-ishihara**
**Chofu-shi Tokyo-to (JP)**

(74) Representative: **Watkins, Arnold Jack et al**
**European Patent Attorney Frank B. Dehn & Co.**
**Imperial House 15-19 Kingsway**
**London WC2B 6UZ (GB)**

The file contains technical information
submitted after the application was filed and
not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a vaccine for preventing or inhibiting human dental caries induced by cariogenic *Streptococcus mutans*.

Dental caries is a disease of teeth which is directly induced by oral bacilli and primarily occurs on the surface of a tooth and progressively breaks down its structure. Among cariogenic oral bacilli, *Streptococcus mutans* is believed to be most important. It has, hitherto, been disclosed, for example, in Bergy's Manual of Determinative Bacteriology, page 502 (1974) that a relationship exists between dental caries and *S. mutans*, it being suggested that *S. mutans* is a similar organism to *S. salivarius*, even though *S. mutans* has not yet been extensively studied and compared with *S. salivarius*. Nowadays, however, *S. mutans* which produces dextran-like polysaccharides from sucrose is clearly distinguished from *S. salivarius* which produces fructans from sucrose.

It is well known that strains of *S. mutans* adhere to the surface of the teeth of humans and animals and form dental plaque comprising insoluble dextran-like polysaccharides (hereinafter referred to as DPS) which are solid and adhesive. The major proportion of *S. mutans* in the oral cavity resides in the plaque to produce lactic acid which directly breaks down the teeth. Thus, for example, it is said that the main constituents of the insoluble and water-soluble DPS produced by *S. mutans* from sucrose are high molecular weight polymers of glucose, comprising respectively $\alpha(1\rightarrow3)$ and $\alpha(1\rightarrow6)$ bondings [Guggenheim, Internat. Dent. J., 20:657—678 (1980)] and British Patent 1,375,866 also discloses that the formation of pre-carious dental plaque is a prerequisite for the development of dental caries and that glucosyltransferase is also an important factor in the development of dental caries.

It is known that dental caries induced by *S. mutans* may be controlled by using mutant strains of *S. mutans*. U.S. Patent 4,133,875 of Hillman (1979) discloses a process for controlling dental caries by injecting an inoculum of a purely cultured *S. mutans* JH 140 (ATCC 31341) or JH 145 (ATCC 31377) into the oral cavity, said strains being isolated from colonies obtained by culturing BHT-2 (str.), a streptomycin-resistant mutan strain of *S. mutans* in a glucose-tetrazolium medium having *inter alia* the following characteristics:—

(a) inability to produce detectable lactic acid when incubated in the presence of glucose;

(b) relatively better adhesion to hydroxyapatite than the parent strain; and

(c) higher productivity of plaque than the parent strain when incubated in the presence of sucrose.

However, JH140 and JH145 did not give good results when used in practice for controlling human dental caries and, by way of example, the following problems arose:—

(1) *S. mutans* is classified into certain strain types. As is well known, Bratthall classified *S. mutans* into 5 serotypes ('a' to 'e') in view of the immunological specificities of the polysaccharides contained in the cell wall [Odont. Revy., 20:141 (1970) and ibid. 20:23 (1970)], to which two serotypes ('f' and 'g') were added later [Perch et al, Acta. Path. Microbiol. Scand. Section B, 82:357 (1974)]. Makoto Sato, one of the coinventors of this invention, has classified *S. mutans* into Human I (HI), Human II (HII) and Rat (R) types with regard to the specific antigens of *S. mutans*, which respectively correspond to said "'c', 'e' and 'f'", "'d' and 'g'" and "'a' and 'b'" serotypes, and has reported:—

(a) 93.9% of *S. mutans* of human origin were Human I type and the remaining strains were Human II type;

(b) all strains of rat origin were Rat type;

(c) no *S. mutans* was isolated from mice and guinea pigs; and

(d) all strains originating from hamsters and monkeys were Human I type. [Journal of Dental Health in Japanese version, Vol. 28, No. 2, pages 100—123 (1978)].

BHT-2, the parent strain of JH140 and JH145, is a known strain belonging to the Rat type which differs from the Human type with regard to serological characteristics and which has not yet been isolated from humans.

(2) JH140 and JH145 form a larger amount of plaque and have a higher ability of adherence than their parent strain BHT-2. It is common knowledge in the art that the formation of a large amount of dental plaque and better adherence to the tooth surface results in the induction of dental caries. Although the Hillman strains are deficient in cariogenic factors because of their low lactic acid productivity other oral bacilli produce lactic acid. The reason why *S. mutans* is believed to be most important among cariogenic bacilli is that insoluble DPS binds *S. mutans* to the surfaces of the teeth so that lactic acid is accumulated on the teeth.

(3) It is not clear whether or not JH140 and JH145 are capable of inhibiting the growth of other cariogenic strains of *S. mutans*.

(4) In order to use living strains for humans, it is necessary to show that they are genetically stable and absolutely safe to human health. It is not clear whether JH140 and JH145 revert to the parent strain, but no cariogenic mutant strain appears to arise from Hillman's strains.

Although *S. mutans* is highly mutative and has been classified into certain types, the relationship between various mutant strains of different types has not, hitherto, been sufficiently clarified. In order to clarify this question, the coinventors of this invention produced a number of mutant strains from various known wild strains of *S. mutans* and investigated their colonial characteristics, productivities of insoluble and water-soluble DPS, cariogenic potentials and other biological characteristics. As a result, the following three mutational phases of *S. mutans* were found regardless of the known serotypes and biotypes of *S. mutans*.

(1) Phase I

"Normal Phase" widely distributed in the oral floras of humans and animals. When cultured on a sucrose-containing agar plate medium such as, for example, TYC agar medium [Stoppelaar et al, Arcs. oral Biol., 12, 1199—1201 (1967)], solid colonies covered with a large amount of insoluble DPS are formed. Each colony has an irregular and raised margin and its central area is rough. There are minor morphological differences between respective colonies. The plaque formed is abundant, solid and adhesive and adheres onto the tube wall and the tooth surface. The productivity of lactic acid is high, and the cariogenicity is the highest of all 3 phases.

(2) Phase II

An essentially unstable phase. As there is a mutual variability between Phases I and II, this phase may sometimes occur by subculturing Phase I. When cultured on a sucrose-containing agar plate medium, round colonies like water-drops are formed, which are bright and somewhat mucoid. There are considerable morphological differences between respective colonies. The productivity of lactic acid and the cariogenicity are weaker than those of Phase I.

(3) Phase III

Obtainable by artificial induction from Phase I, for example, by using nitrogen mustard, nitrosoguanidine, or irradiation by ultraviolet rays. Various characteristics of this phase are entirely different from those of other phases. When cultured on a sucrose-containing agar plate medium, the colonies formed are round, bright, opaque, smooth and large. In the test tube, the productivity of lactic acid is low. The productivities of insoluble and water-soluble DPS and the ability to form wire plaque are substantially deficient. Thus, the cariogenic potential is also substantially reduced. Animal tests also confirm that the ability to form plaque and the cariogenic potential are substantially reduced.

Moreover, further studies reveal the following interesting biological activity of Phase III.

This phase is capable of specifically inhibiting the growth of the cariogenic wild strains of at least Human type *S. mutans* in coexistence with them *in vitro* and *in vivo* so that the induction of dental caries by *S. mutans* may be completely prevented or at least inhibited. Where Phase III is inoculated into a culture or human oral cavity where the plaque has already been formed by the action of Phase I strains, Phase III intrudes compulsively into such plaque to "reside" there, and Phase I strains are outwardly destroyed and gradually disappear. The plaque is broken down and the Phase III strains then die owing to the loss of their support medium or residence and their inability to form new plaque. Where Phase I strains are introduced into a test tube or oral cavity already occupied by a Phase III strain, the Phase I strain dies. Such unique biological activity has also been confirmed by human and animal tests.

Various characteristics of Phase III strains are genetically stable. For example, no reversion to the parent strain was observed nor was there any observable generation of any cariogenic mutant strain when subculturing was effected on various known media e.g. TYC agar media and no such effects were observed when Phase III strains were continuously administered to hamsters, both subculturing and continuous administration being effected over an extended period of time (more than 6—12 months).

Phase III strains have been artificially produced by the inventors from the cariogenic wild strains of *S. mutans* (hereinafter referred to as artificial Phase III strains). However, in order to obviate the need for further processing which would otherwise be necessary to enable administration of Phase III strains directly and safely into the oral cavity of humans Phase III strains were isolated from humans (hereinafter referred to as natural Phase III strains) and the artificial and natural Phase III strains were compared with each other. Very many strains of *S. mutans* were collected from more than 1000 adults and infants. It has been unexpectedly found that wild strains of Human I and II types *S. mutans* having the characteristics of Phase III strains are living in the oral floras of young humans, particularly infants.

They were isolated and purely cultured. It has also been found that the hosts of natural Phase III strains experience very little, if any, dental caries and the cariogenic wild strains of *S. mutans* do not appear to be present in coexistence with the natural Phase III strains. In this regard, it should be appreciated that virulent oral bacilli other than *S. mutans* do exist and may be present even though their cariogenic potentials are weaker than *S. mutans*. The reason why natural Phase III strains exist in the oral floras of infants and children is not yet clear.

It has been found that the biological characteristics of artificial and natural Phase III strains are quite similar to each other, as exemplified in the following Tables 1—6. In these tables, the enzymatic activities were determined by the Inoue et al method [J. Dental Health, 24, 6—18, 1970 in Japanese version], protein N was determined by the E. F. Hartely method [Anal. Biochem., 48, 422, 1972] and the agglutination titre was determined by the Sato method [Makoto Sato, J. Dental Health, 28, 2, 99, 1978 in Japanese version].

Table 1 (Decomposition of sugars) shows artificial Phase III strains produced by using the well-recognized reference strains of *S. mutans* as follows:— HS-1 (a); FA-1 (b); Ingbritt (c); NCTC 10449; ATCC 25175 (c); OMZ176 (d); P-4 (e); OMZ175 (f); KIR (g); and RC-20 (b). The serotypes are indicated in the brackets. Other decompositions are the same as the decompositions of NCTC 10449, a typical cariogenic

EP 0 090 617 B1

wild strain of *S. mutans* (Phase I). The microorganism NCTC 10449 is deposited with the National Collection of Type Cultures, Central Public Health Laboratory, 175 Colindale Avenue, London NW9 5HT, England and with the American Type Culture Collection (ATCC), 12301 Parklawn Drive, Rockville, Maryland 20852, U.S.A. as ATCC 25175.

TABLE 1
Decomposition of sugars (artificial Phase III)

| Sugar | Phase I | II | III |
|-------|---------|-----|-----|
| Raffinose | −~+ (1) | − | + |
| Salicin | − | −~+ (4) | + |
| Cellobiose | −~+ (2) | − | + |
| Mellibiose | −~+ (3) | − | + |
| Esculine* | + | + | +~− |

Notes:—
*Generally weak and may vary with differing the individual strains.
(1)+ Ingbritt; (2)+ 10449, OMZ176, & RC-20
(3)+ Ingbritt, (4)+ HS-1, P-4 & RC-20

Table 2 shows (A) sucrase activity ($\times 10$ U/mg of protein N), (B) glucosyltransferase activity (U/mg of protein N), (C) invertase activity ($\times 10$ U/mg protein N) and (D) protein N (mg/ml) of the reference strains shown in Table 1 as well as of their artificially induced Phase III strains. In this table, Human I and II types are marked with * and ** respectively.

4

# EP 0 090 617 B1

### TABLE 2
### (Enzymatic activities and protein N)

A: sucrase (total) ($\times$10 U/mg protein N), B: glucosyltransferase ($\times$10 U/mg protein N), C: invertease ($\times$10 U/mg protein N), *: Human I type, **: Human II type, ( ): serotype, D: protein N (mg/ml)

| Strain | Phase | A | B | C | D |
|---|---|---|---|---|---|
| HS-1(a) | I | 469 | 148 | 321 | 0.027 |
| | III | 25.3 | 11.2 | 14.1 | 0.031 |
| | % | 5.39 | 7.57 | 4.39 | 114.8 |
| FA-1(b) | I | 476 | 394 | 82 | 0.021 |
| | III | 17.4 | 7.6 | 9.8 | 0.029 |
| | % | 3.66 | 1.93 | 11.95 | 138.1 |
| Ingbritt(c)* | I | 484 | 235 | 239 | 0.0127 |
| | III | 49.4 | 41.4 | 8.0 | 0.017 |
| | % | 10.21 | 17.62 | 3.35 | 133.9 |
| NCTC10449(c)* | I | 915.8 | 380.1 | 535.7 | 0.013 |
| | III | 23.7 | 13.3 | 10.4 | 0.026 |
| | % | 2.59 | 3.50 | 1.94 | 200.0 |
| OMZ176(d)** | I | 498 | 388 | 232.1 | 0.015 |
| | III | 55.6 | 23.2 | 37.6 | 0.027 |
| | % | 11.16 | 5.98 | 16.20 | 180.0 |
| P-4(c)* | I | 449 | 148 | 311 | 0.016 |
| | III | 57.6 | 38.7 | 19.0 | 0.021 |
| | % | 12.83 | 26.15 | 6.11 | 131.3 |
| OMZ175(f)* | I | 432 | 188 | 245 | 0.024 |
| | III | 46.7 | 21.2 | 25.4 | 0.033 |
| | % | 10.8 | 11.28 | 10.4 | 137.5 |
| KIR(g)** | I | 373 | 232 | 152 | 0.028 |
| | III | 43.4 | 16.7 | 27.8 | 0.031 |
| | % | 11.64 | 7.20 | 18.29 | 110.7 |
| RC-20(b) | I | 544.3 | 259.2 | 285.1 | 0.022 |
| | III | 16.8 | 4.2 | 12.5 | 0.032 |
| | % | 3.09 | 1.62 | 4.38 | 145.5 |

Table 3 shows the maximum, minimum and average values of the enzymatic activities and reduction ratios (parent=100%) collected from Table 2.

### TABLE 3

| Human type | | Sucrase | Glucosyl-transferase | Invertase |
|---|---|---|---|---|
| Activity | max. | 57.6 | 41.4 | 37.6 |
| | min. | 23.7* | 13.3* | 8.0 |
| | average | 46.1 | 25.8 | 23.45 |
| Reduction % | max. | 12.8 | 17.6 | 18.29 |
| | min. | 2.57* | 3.50* | 1.94* |
| | average | 9.87 | 11.96 | 10.11 |

Note:—
*Phase III strain induced from NCTC10449.

The following Tables 4 to 6 correspond to Tables 1 to 3 respectively to indicate the numerical values of natural Phase III strains which were isolated by the present coinventors from the human oral floras. Table 4 also shows the agglutination titres. The numerical values of NCTC 10449 shown in Table 2 are inconsistent with those shown in Table 5 as the measurements were not effected at the same time.

TABLE 4
Natural Phase III strains
Ag: agglutination titres,
Ma: mannitol, So: sorbitol, Ra: raffinose, Sa: salicin,
Ce: Cellobiose, Me: Mellibiose, La: lactase,
Su: sucrose, Es: esculine

| Strain TMD-NC | FERM- | Ag | Ma | So | Ra | Sa | Ce | Me | La | Su | Es |
|---|---|---|---|---|---|---|---|---|---|---|---|
| Inoue | | 256 | + | + | + | + | + | + | + | + | + |
| T. Komatsu | | 2048 | + | + | + | + | + | + | + | + | + |
| K. Komatsu | | 1024 | + | + | + | − | + | + | + | + | + |
| Kawabe | | 1024 | + | + | + | − | + | + | + | + | − |
| Nakayama | | 1024 | + | + | + | − | + | + | + | + | − |
| Kume* | BP 319 | 1024 | + | + | + | − | + | − | + | + | + |
| 26P | P 6876 | 256 | + | + | + | + | + | + | + | + | + |
| 49 | BP 318 | 256 | + | + | + | + | + | + | + | + | − |
| 57-4 | P 6878 | 1024 | + | + | + | + | + | + | + | + | + |
| NCTC10449** | | 2048 | + | + | − | − | + | + | + | + | + |

Notes:—
   *All strains are Human I type (serotype 'c') except Kume (Human II type, serotype 'd').
   **Other decompositions are the same as those of NCTC 10449 (Phase I) otherwise specified.

TABLE 5
Enzymatic activities of natural Phase III strains
A: Sucrase (total), B: Glucosyltransferase, C: Invertase

| Strain TMD-NC | FERM- | A | B | C |
|---|---|---|---|---|
| | | (Unit: $10^2$ U/mg protein N) | | |
| Inoue | | 0.57 | 0.28 | 0.28 |
| | | 10.69 | 9.30 | 12.07% |
| K. Komatsu | | 0.44 | 0.16 | 0.28 |
| | | 8.26 | 5.32 | 12.07% |
| T. Komatsu | | 0.39 | 0.15 | 0.23 |
| | | 7.32 | 4.98 | 9.91% |
| Kawabe | | 0.56 | 0.25 | 0.31 |
| | | 10.51 | 8.31 | 13.36% |
| Nakayama | | 0.46 | 0.25 | 0.20 |
| | | 8.63 | 8.31 | 8.62% |
| Kume** | BP 319 | 0.35 | 0.14 | 0.24 |
| | | 6.57 | 4.65 | 10.34% |
| 26P | P 6876 | 0.43 | 0.17 | 0.28 |
| | | 8.07 | 5.65 | 12.07% |
| 49 | BP 318 | 0.37 | 0.17 | 0.20 |
| | | 6.94 | 5.65 | 8.62% |
| 57-4 | P 6878 | 1.30 | 0.29 | 1.00 |
| | | 24.40 | 9.63 | 43.10% |
| NCTC10449* | | 5.33 | 3.01 | 2.32 |
| | | 100.00 | 100.00 | 100.00% |

*Control (Phase I strain).
**Human II type. Other strains are Human I type.

TABLE 6

| | | Sucrase | Glucosyl-transferase | Invertase |
|---|---|---|---|---|
| Activity | max. | 1.30 | 0.29 | 1.00 |
| | min. | 0.35 | 0.14 | 0.20 |
| | average | 0.54 | 0.21 | 0.34 |
| Reduction % | max. | 24.40 | 9.63 | 43.10 |
| | min. | 6.57 | 4.65 | 8.62 |
| | average | 10.15 | 6.87 | 14.46 |

Note:— Reduction ratio: NTCC 10449=100%

From Tables 1—6, the following observations may be noted.

(1) There are no significant differences between the characteristics of the Phase III strains shown in these tables.

(2) With regard to the drastic reduction in enzymatic activities from Phase I to Phase III, it is believed that glucosyltransferase is the enzyme which directly participates in the induction of dental caries induced by *S. mutans*.

(3) The artificial Phase III strain obtained from NCTC10449 and the natural Phase III strain identified as TMD-NC 57-4 show the highest reduction ratios of enzymatic activities. The artificial Phase III strain from Ingbritt (A=4.9; B=4.14) and OMZ175 (C=25.4) and the natural Phase III strain designated as TMD-NC 57-4 show the highest activities. However, all Phase III strains exemplified in these tables completely inhibited the growth of the cariogenic wild strains of *S. mutans* and their plaque-forming ability was also substantially reduced, as confirmed by *in vitro* and *in vivo* tests.

(4) Although the decomposition spectra of Phase III are broader than those of other phases, it is difficult to characterize Phase III by the decomposition of sugars.

It is known that *S. mutans* has a cariogenic potential, but the present invention is based on the discovery that certain mutant strains of *S. mutans* have a substantially reduced cariogenic potential and are moreover capable of inhibiting the growth of the cariogenic wild strains of *S. mutans in vivo* and *in vitro* regardless of the type of cariogenic strain. The present invention is also based on the discovery that such mutant strains may be formulated into an inoculate administration to the oral cavity for preventing or inhibiting *Streptococcus mutans* induced dental caries.

Thus according to one feature of the present invention there is provided an inoculate for preventing or inhibiting *Streptococcus mutans* induced dental caries characterized in that said inoculate comprises as active ingredient living cells of a naturally occurring human type mutant strain of *Streptococcus mutans*, the mutant strain being effective to inhibit the growth of cariogenic wild strains of *Streptococcus mutans* and edible lactic acid-producing bacteria and being substantially deficient in cariogenic potential in the human oral cavity.

The term "inoculate" as used herein means a composition for administration to the oral cavity, which composition is capable of preventing or inhibiting plaque formation and dental caries induced by cariogenic wild strains of *Streptococcus mutans*. The term "inoculate" as used herein thus includes not only freeze-dried formulations and physiologically acceptable solutions e.g. in which the living cells are presented in association with a physiologically acceptable liquid carrier, but also food compositions which comprise living cells of the mutant strains hereinbefore defined. It will be understood that the food composition may be liquid, solid or semi-solid as, for example, in milk, cheese, pickles or yogurt. Where the inoculate is presented in the form of a liquid or semi-solid food composition comprising a liquid or semi-solid physiologically acceptable carrier the concentration of living cells of the mutant strain in the composition is preferably from $10^5$ to $10^8$ cells/ml of the carrier.

The mutant strain of *S. mutans* employed as active ingredient has a substantially reduced ability to produce dextran-like polysaccharides and is substantially deficient in cariogenic potential in a hamster's oral cavity.

The mutant strain is effective to specifically inhibit the growth of cariogenic wild strains of *S. mutans*.

The mutant strain of *S. mutans* may if desired be isolated as a wild strain from the human oral cavity. Such mutants are advantageously isolated from the oral flora of children and include for example *Streptococcus mutans* TMD-NC Kume (FERM-BP 319), *Streptococcus mutans* TMD-NC 26P (FERM-P 6876), *Streptococcus mutans* TMD-NC 49 (FERM-BP 318) or *Streptococcus mutans* TMD-NC 57-4 (FERM-P 6878). The above-mentioned mutant strains have all been deposited with Bikoken (as hereinbefore described), the microorganisms being filed on 21st January 1983.

The mutant strains are preferably pure cultured for use in the inoculates of the present invention. A non-cariogenic drink or liquid or semi-solid food composition comprising the living cells obtained from a purely cultured mutant strain of Human type *S. mutans* and the living cells of edible lactic acid-producing bacilli known *per se* in association with a liquid carrier, constitutes a further embodiment of the present invention.

The living cells of the non-cariogenic mutant strain and the lactic acid-producing bacilli are preferably dispersed in the liquid carrier to form for example a drink or yogurt-like composition.

Such non-cariogenic compositions may for example be used to prevent or inhibit human dental caries completley and simply.

Any mutant strain of a Human type (as hereinbefore defined) *S. mutans* which specifically inhibits the growth of the cariogenic strains of *S. mutans* and which is substantially deficient in cariogenic potential in the human oral cavity or, as described in the following experiments, in a hamster's oral cavity may be used for the purpose of this invention, and thus the strains which may be used are not limited to those exemplified in the foregoing tables. In any event, the use of mutant strains other than Human type strains as hereinbefore defined should be avoided in view of the effects which it is desired to obtain and in view of safety considerations. The use of a pure culture, is preferred since it is then no longer necessary to process the strains further, for example, in the preparation of dental inoculates.

It is preferred to use a mutant strain which has a substantially reduced ability to produce DPS as hereinbefore defined and a substantially reduced ability to form plaque *in vitro* because such a strain has an especially reduced cariogenic potential in the human oral cavity.

In practice, it is advantageous to use a mutant strain having the morphological characteristics of Phase III strains as follows:— The characteristics of such strains are similar to those of NCTC 10449, a typical wild strain of the cariogenic *S. mutans*, otherwise specified.

I. Morphology:
Streptococcus, 1 μ×1—2 μ, Gram-positive.

8

II. Growth on various media [37°C; 24 hours; specified pH]:

(1) TYC agar plate medium [Stoppelaar et al, Archs. oral Biol., 12, 1199—1201, 1967]:

Insoluble and water-soluble DPS are not produced. Colonies are round, flat, white, opaque, bright and large.

(2) Todd Hewitt broth agar medium [Baltimore Biological Laboratories, Inc., U.S.A., hereinafter referred to as BBL]:

Colonies are round, flat, opaque, bright, somewhat mucoid and large.

(3) Mitis-salivarius agar plate medium (Difco., U.S.A.):

DPS are not produced. Colonies are round, pale whitish, somewhat mucoid and large.

(4) Brain Heart Infusion agar plate medium (BBL):

Colonies are round, somewhat whitish, opaque and large.

(5) Tryptocase soy broth medium (BBL):

Growing from the lower layer of the medium.

(6) Todd Hewitt broth (BBL):

Growing from the lower layer of the medium.

III. Physiological characteristics:

(1) Productivity of wire plaque (showing the adhering ability by insoluble DPS): negative.

(2) Agglutination of cells by DPS: negative [determined by Freedman et al method (Infect. Immun., 10, 189—196, 1974) using Dextran T250, commercial product of Pharmacia Fine Chemicals AB., Sweden].

(3) Formation of pigment: negative.

(4) Growth range: pH . . . 5—8, temperature . . . 10—39°C.

(5) Decomposition of sugars: as shown in Table 4.

IV. Miscellaneous:

Hydrolysis of esculin . . . positive.

Hemolysis (sheep) . . . −~+.

Glucosyltransferase activity . . . substantially deficient.

Productivity of lactic acid . . . very weak.

Ability of infection to animals . . . negative.

V. Cariogenicity .. substantially deficient; Human I or II type.

VI. Stability:

Where, for example, the strain was subcultured over an extended period of time (more than 6 months) on TYC agar plate media (pH=about 7.3; 37°C; 24 hours) or treated with known mutagens such as, for example, nitrogen mustard, nitrosoguanidine, irradiation by ultraviolet ray and the like, no revertion, or any generation of cariogenic mutant strains was noted. Generation of new dental caries, ingravescence of old caries, increase in the antibody titres in blood and saliva and various other undesired side effects were not found to any significant degree on administering the living cells to humans and animals continuously over an extended period of time.

VII. Interference between Phase III and other phases:

Phase III strains specifically inhibit the growth of the cariogenic strains of S. mutans (Phases I and II) in vitro and in vivo.

The non-cariogenic mutant strains which may be used for the purpose of this invention may be cultured in conventional manner as in the case of known S. mutans. Thus, the culturing may be effected aerobically, although the anaerobic culturing is preferred. Although both organic and synthetic media may be used, liquid media may be preferred for mass propagation. Various media suitable for culturing streptococci may be used at a pH of 5.6—9.0, for example, about 7.0 and at a temperature of 23—39°C, for example, 37°C. The acid production is weak so that the pH of the strain medium usually decreases to 5.2—5.6 within 48 hours from the beginning of the fermentation. After completion of the culturing, the cultured broth contains, for example, about $10^8$ cells/ml.

It is possible to administer the cultured liquor to the human oral cavity or to add the cultured liquor to an appropriate liquid carrier known per se for example in the preparation of various liquid food compositions e.g. drinks containing the living cells of edible lactic acid-producing bacilli such as yogurts. However, it is preferred to separate the living cells from the cultured broth in conventional manner (e.g. by centrifugation, 8000 r.p.m./20 min.) and suspended the same in an appropriate liquid carrier such as, for example, water, syrup, animal milk etc. It is also preferred to freeze-dry the separated cells in conventional manner and divide them into appropriate dosage unit forms for preservation, if desired, in association with a suitable preservative such as, for example, milk sugar, or gelatin. For use, the living cells may preferably be dispersed in a suitable liquid carrier. The freeze-drying and preservation may be effected in conventional manner.

It has been found that the non-cariogenic mutant strains of S. mutans of this invention intrude specifically into the plaque formed by the cariogenic S. mutans strains (Phase I) as they are incapable of forming plaque themselves. Thus, they reside in the plaque until the plaque is completely destroyed. Where plaque is absent, the non-cariogenic mutant strain of this invention is unable to find a support medium in the oral flora and they will be killed in a relatively short period of time as they are not capable of forming plaque themselves and moreover their ability to adhere is essentially weak. Thus, it is preferred to administer a large amount of the non-cariogenic mutant strain to the human oral cavity. For example, in the case of administration at a daily dose of about $10^5$ to $10^7$ cells/ml of the liquid carrier once after meals, the cariogenic wild strains of *S. mutans* may disappear from the oral cavity for example in one or two weeks after the beginning of administration. It is also possible, if desired, to administer the living cells of the non-cariogenic mutant strain of this invention in a larger amount (e.g. about $10^8$ cells/ml or more).

It may indeed be possible to admix the living cells of the non-cariogenic strain of this invention with foodstuffs containing the living cells of edible lactic acid-producing bacilli such as e.g. cheese or pickles. However, it is especially advantageous to mix the non-cariogenic strain of this invention with a drink known *per se* containing the living cells of edible lactic acid-producing bacilli whereby to prevent or inhibit dental caries easily and comfortably. Various animal tests have revealed moreover that the cariogenicity of the known edible lactic acid-producing bacilli may be completely prevented or inhibited by using such a mixture without any significant side effects.

Lactic acid-producing bacilli denote the bacilli which produce lactic acid from sugars and are classified into Gram-positive bacilli such as streptococci and Gram-negative bacilli such as lactobacilli. Various drinks containing the living cells of such bacilli have been marketed for many years in various countries of the world owing to a belief that they are biologically active against the growth of harmful microorganisms in the intestinal flora of humans. The lactic acid-producing bacilli are usually cultured purely in view of their severe nutrient-requirements and are used, for example, in the form of drinks comprising an animal milk (e.g. cow milk) for dispersing the bacilli. However, it is well known that such bacilli have a cariogenic potential. For example, British Patent 1,375,866 discloses the *Lactobacillus acidophilus, L. casei, Streptococcus faecalis* etc. are well known aetiological factors, and Oshima [J. oral Health, Vol. 16, No. 1, pages 161—169, 1978 in Japanese version] reported that *Lactobacillus casei* and *L. helvetius* used for yogurts commercially available in Japan induced dental caries.

All edible lactic acid-producing bacilli conventionally used for the preparation of various drinks containing the living cells of such bacilli may be used for the purpose of this invention. They are exemplified by *Lactobacillus bulgaricus, L. acidophilus, Streptococcus lactis, S. thermophilus, L. casei, L. bifidus, L. helvetius* etc. They are usually cultured separately and used solely or in combination, for example, *L. bulgaricus* with *S. lactis*; *S. thermophilus* with *L. bulgaricus*; *L. casei* with *L. helvetius* etc.

According to an ordinance issued by the Ministry of Welfare and Public Health of the Japanese Government, entitled Specifications of milks and milk products, under the Food Sanitation Law of Japan, the lactic-acid producing bacilli designated by law are such bacilli that are capable of forming yellowish colonies by culturing a sample on a bromocresol purple-containing plate counter medium under specified conditions (e.g. 35—37°C; 72 hours). As the non-cariogenic mutant strains of this invention fall completely within this requirement, it is possible to add the non-cariogenic mutant strains of the present invention to the known drinks containing edible lactic acid-producing bacilli. In this case, the concentration of the non-cariogenic mutant strain of this invention to be added may be the same as that for use alone.

For the purpose of this invention, the inoculate of the invention is advantageously formulated into a yogurt which is especially preferred amongst various drinks and foodstuffs containing the living cells of edible lactic acid-producing bacilli. The preparation of yogurt is well known in the art and comprises, in general, sterilizing milk (e.g. cow's milk, goat's milk, buffalo milk etc. which may be in the form of skim milk, reduced milk, reduced skim milk, homogenized whole milk and the like), adding to the sterilized animal milk a starter (pure culture of lactic acid-producing bacilli) and culturing the same at a suitable temperature to obtain a solid curd. Commercially available yogurts may be classified into sour plain yogurts and fruit yogurts containing a small amount of milky solids and a hardening agent. More specifically, yogurt may be produced, for example, by concentrating a whole milk *in vacuo* to reduce its volume to 2/3 or mixing a whole milk with a skim milk, adding, if desired, to the solution sucrose (8—9%), agar etc. to give an adjusted solid contents (12—14%), sterilizing the mixture at 85—90°C for 30 minutes, cooling the mixed solution to 33—40°C, adding a starter (2—2.5%) to the solution with stirring, putting the mixture into a yogurt bottle, culturing the starter at a suitable temperature (e.g. 33—47°C) for a suitable time (e.g. 10—15 hours) to give an acidity of 0.8—1.2, discontinuing the fermentation and preserving the bottle at a cool temperature. In another example, yogurt may be prepared by adding a suitable amount of 5% glucose or sucrose solution to a skim milk to give 10% solids, adding a starter (2—2.5%) to the mixture, culturing the starter at a suitable temperature for a suitable time (e.g. 24—72 hours) to give an adjusted acidity of 0.8—1.2%, discontinuing the fermentation, homogenizing the cultured borth with agitation, and adding a 10% sucrose solution to the fermented liquor. Suitable sweetening agents, essences preservatives etc. may be added to the product. It is also known to replace at least part of the animal milk by soybean milk. In general, yogurts commercially available in Japan contain milky solids (12—14%), sucrose (6—8%), lactic acid (1%) and the living cells of the lactic acid-producing bacilli (about $10^8$ or more/ml). Where the non-cariogenic mutant strains of this invention are added to yogurt and the like having similar contents, it is sufficient to add the non-cariogenic mutant strain of this invention at a concentration of about $10^5$—$10^7$ cells/ml. In such a case, it is possible to completely prevent or inhibit dental caries induced by cariogenic strains of *S. mutans* and lactic acid-producing bacilli without any significant side effects upon the quality of the drink *per se*. Although it is possible if desired to use a larger amount of the non-cariogenic mutant strain of this invention, the use of an excessively low concentration of the same should be avoided in view of its poor adhering ability.

For example, where a drink containing about $10^7$ to $10^9$, e.g. $10^8$ cells of conventional lactic acid-producing bacilli and about $10^6$ living cells of the non-cariogenic mutant strain of this invention per ml was administered to humans at a dose of 50—100 ml once daily after meals, the concentration of the cariogenic wild strains of *S. mutans* decreased continuously and finally disappeared from the oral cavities about 1—2 weeks after the beginning of administration. Various experiments using humans and hamsters have revealed that dental caries induced by *S. mutans* and lactic-acid-producing bacilli may be completely prevented or inhibited by using the inoculate of the present invention in the form of a mixed drink. The reason for such effects is not yet clear.

It is possible to add various known additives such as, for example, preservatives, hardening agents, essences and the like to the mixed drink of this invention.

After being preserved for 10 days at a low temperature (e.g. not higher than 10°C) the number of living cells of the non-cariogenic mutant strain and lactic acid-producing strain in the mixture as well as other properties of the drink were substantially unchanged. The mixed cells of this invention do not exert any adverse influence upon the flavour and taste inherent in the drink containing lactic acid-producing bacilli. Thus, the mixed drink of this invention exhibits synergistic effects obtained by the combined use of the non-cariogenic strains of the present invention and conventional lactic acid-producing strains.

It is possible to prepare a mixed drink according to this invention by using the living cells of the non-cariogenic mutant strains of Human I and II types either alone or in combination. With regard to the inhibition of the growth of the cariogenic wild strains, the existence of a significant specificity is observed between the Rat type strains and the Human type strains. However, such a significant specificity was not observed between the strains of Human I and II types.

According to a further aspect of this invention, there is provided a dental inoculate comprising the living cells of the non-cariogenic mutant strain of *S. mutans* as hereinbefore defined.

This type of live inoculate for preventing or inhibiting dental caries induced by *S. mutans* was not previously known. The live inoculate of this invention may be prepared in conventional manner. For example, the living cells of the non-cariogenic mutant strain of this invention are separated from the cultured broth by centrifugation and suspended in a suitable buffer solution. The cells are washed by centrifugation and diluted with an appropriate buffer solution to an appropriate concentration (about $10^8$ cells/ml at pH 6.8—7.0), and gelatin, lactose and the like are added as preservatives. The mixture is divided into small fractions and placed into vials, followed by freeze-drying to obtain a live vaccine. For use, the inoculate is diluted with a suitable physiologically acceptable solution or with pure water to a concentration of about $10^6$—$10^7$ living cells/ml. This inoculate may be administered, for example, into the oral cavity of humans at a daily dose of 0.1—1 ml/once before bedtime. Usually, the inoculation may be effected continuously for 3—5 days. In this manner, it is possible to prevent or inhibit human dental caries induced by *S. mutans* with good results.

In the following non-limiting Examples, which illustrate the present invention, and in the following experiments, the culturing was effected in a nitrogen atmosphere (90%) containing $CO_2$ gas (5%) and hydrogen gas (5%):—

Example 1

A 5% glucose solution containing skim milk (10% as solids) is sterilized at 110°C for 15 minutes. To this solution, *Lactobacillus casei* is inoculated and anaerobically cultured in conventional manner at 37°C for 36 hours. The cultured liquor is homogenized with agitation to obtain a first solution. Separately, *Streptococcus mutans* TMD-NC 49 (FERM-BP 318) is cultured in a similar manner to that described above and the culture liquor is homogenized to obtain a second solution. To a 10% fructose solution, the thus-obtained first and second solutions are added to obtain a mixed drink of this invention which contains about $10^8$ living cells of *L. casei* and about $10^6$ living cells of TMD-NC 49 per ml. Separately, the first solution is added to a 10% fructose solution to obtain a control drink which contains about $10^8$ living cells of *L. casei* per ml. In comparison with the control drink, the mixed drink of this invention has an excellent flavour and a better taste.

Example 2

*Streptococcus mutans* TMD-NC 49 (FERM-BP 318) cultured anaerobically at 37°C for 36 hours on Todd Hewitt broth medium (pH 7.2). The living cells are separated from the cultured broth by centrifugation (8000 r.p.m./20 min.). The cells and the first solution obtained by the method of Example 1 are added to a 10% fructose solution to obtain a mixed solution containing about $10^8$ and about $10^6$ cells/ml of *L. casei* and *S. mutans* per ml respectively. Similarly, a control drink containing *L. casei* alone is prepared. In comparison with the control drink, the mixed drink exhibits a very nice flavour.

Experiments

In the following experiments, the following materials were used, to which additives such as essences, hardening agents, preservatives and the like were conveniently not added.

(1) Material I corresponds to the control drink of Example 1 and contains the living cells of *Lactobacillus casei* (about $10^8$ cells/ml). Material II corresponds to the mixed drink of Example 2 and contains the living cells of *L. casei* (about $10^8$ cells/ml) and *Streptococcus mutans* TMD-NC 49 (FERM-BP 318); (about $10^6$ cells/ml). Material III was prepared by culturing *Streptococcus mutans* NCTC10449, a typical cariogenic wild strain by the method of Example 2, separating the cells from the cultured broth and adding the same to Material I to obtain a cariogenic drink containing about $10^8$ living cells of *S. mutans* NCTC 10449 and about $10^8$ cells of *L. casei* per ml.

(2) As test animals, Golden hamsters (each group consisting of 10 hamsters otherwise specified) were used and bred on a cariogenic diet (Diet 2000, Funabashi Nojo, Japan) *ad libitum*.

(3) In the experiments hereinafter described, the lactic acid-producing microorganism used was *Lactobacillus casei*. Although various tests were effected by using *Lactobacillus acidophilus, L. bulgaricus, L. bifidus, Streptococcus thermophilus*, and *S. lactis*, no significant difference was observed in so far as the prevention and inhibition of dental caries induction was concerned.

Experiment 1

Material I (*Lactobacillus casei*) and II (L. casei and *Streptococcus mutans* TMD-NC 49; FERM-BP 318) were administered to hamsters to find out that the cariogenic potential of TMD-NC 49 was negative. The test period was 60 days. From 21 days after birth (weaning period), Material I (0.1 ml once daily) was administered into the buccal cavities of Group I, and similarly Material II was administered to Group II. Samples were optionally collected from the molar surfaces of Group II, and each sample was cultured at 37°C for 48 hours on a TYC agar plate medium (15 ml; pH 7.2) and a Mitis-salivarius agar plate medium (15 ml; pH 7.2; containing 100 µg/ml of bacitracin) to investigate the adherence of TMD-NC 49 to the teeth. Although its adhering ability was lower than that of *S. mutans* NCTC 10449, a typical cariogenic wild strain, TMD-NC 49 adhered to the teeth about 6—12 days after the beginning of administration. After completion of breeding, all animals were anesthetised with pentabarbital and abdominally injected with pilocarpine HCl (0.75%; each 0.1 ml/100 g of body weight). The saliva was collected from each animal which was then killed by cardiac puncture. The maxilla was removed from the animal and treated in an autoclave at 120°C for 1—2 minutes to remove the soft part. The remaining part was washed with water and dried to obtain a sample of the teeth. The results shown in Table 7 were calculated from the numbers of all molars and infected molars of each group, from which it is apparent that the cariogenic potential of *S. mutans* TMD-NC 49 was negative.

TABLE 7

Group I: *L. casei*
Group II; *L. casei* and *S. mutans* TMD-NC 49

|  | Group I | II |
|---|---|---|
| Increased body weight (% in average) | 78.3 | 75.2 |
| Infection rate (all hamsters=100%) | 10. | 0. |
| Infection rate (all molars=100%) | 0.93 | 0. |

Material II [*Lactobacillus casei* and *S. mutans* TMD-49) 0.2 mls/day)] was continuously administered to hamsters for 3 months. The hamsters were then killed and examined pathologically, but nothing unusual was observed.

Experiment 2

Materials II (*Lactobacillus casei* and *S. mutans* TMD-NC 49; FERM-BP 318) and III (*S. mutans* NCTC 10449 and *L casei*) were administered to hamsters to investigate the inhibiting activity of *S. mutans* TMD-NC 49 on the induction of dental caries. The test period was 60 days. Between the 10th and 27th days after birth (teething period of molars), on every other day Materials II and III were in turns administered into the buccal cavities of Group I (test group) at a daily dose of 0.1 ml once. From 21 days after birth, the animals were bred with a cariogenic diet (Diet 2000) alone. Group II was treated in a similar manner to that applied to Group I except that Material II was not used. The third group was bred with Diet 2000 without administering Materials II and III.

After completion of breeding, all molars were investigated in a similar manner to that described in Experiment 1 to obtain the results shown in Table 8, from which the activity of *S. mutans* TMD-NC 49 against cariogenic potential is apparent.

12

TABLE 8

Group I: *L. casei* and *S. mutans* TMD-NC 49 (FERM-BP 318)
Group II: *L. casei* and *S. mutans* NCTC 10449
Group III: Untreated

|  | Group I | II | III |
|---|---|---|---|
| Increased body weight (% in average) | 71.7 | 68.3 | 70.4 |
| Infection rate % (all hamsters=100%) | 20. | 100. | 0. |
| Infection rate % (all molars=100%) | 5. (6/120) | 56.7 (68/120) | 0. |

Experiment 3

Material II (*L. casei* and *S. mutans* TMD-NC 49; FERM-BP 318) was administered to seven members of a family consisting of 8 members including an infant of 4 years old (host of *S. mutans* TMD-NC 49). Before this, samples were collected from their oral floras. Each sample was suspended in a physiological solution of sodium chloride (pH 7.0; 0.2 ml) and subjected to ultrasonic treatment (20 KHz/5—10 min.) to give a precipitate which was then cultured at 37°C for 24 hours on a Mitis salivarius (15 ml; pH 7.2; containing 100 µg/ml of bacitracin) to confirm that the cariogenic wild strains of *S. mutans* ('c' to 'g' serotypes) were living in their oral cavities, confirmation being effected by comparison of the biochemical and serological characteristics of the microorganisms.

Material II (each 50 ml/once daily) was then administered into the buccal cavities of the members of the family every day 3 hours after breakfast. During the test period, no special attention was paid to the members of the family in order to prevent the induction of dental caries except for the use of a commercial tooth paste [White & White, a commercial product of Lion K.K., Tokyo; containing anhydrous silicic acid, wetting agent, bonding agent etc.] twice daily after breakfast and dinner. Optionally, samples were collected from their oral floras and treated in a similar manner to that described above to investigate the disappearance of the cariogenic wild strains of *S. mutans* and the adherence of *S. mutans* TMD-NC 49 onto the teeth. It was found that the cariogenic wild strains disappeared from the oral floras of 2 members of the family 9 days after the beginning of the administration of TMD-NC 49, and from the other members of the family about 2 weeks after the beginning. After disappearance of the wild strains, the administration was continued to all members of the family for 6 months with an interval of 3 days, and further continued to 3 members of the family for an additional 6 months. When the administration was discontinued, *S. mutans* TMD-NC 49 disappeared from the oral floras in a relatively short period of time, although no cariogenic wild strain of *S. mutans* was detected in any members of the family during the administration of *S. mutans* TMD-NC 49.

Experiment 4

A live vaccine obtained by the method of Example 2 was administered into the oral cavities of humans to determine its effects in the following manner.

Dental plaque was collected from 8 persons (adults) and treated as follows on each occasion. The dental plaque was suspended in a 1/75M phosphate buffered sodium chloride solution (pH=7.0; 0.2 ml) and treated with ultrasonic waves (20 KHz; 5—10 min.). The suspension was transferred to a Mitis-salivarius agar plate medium (pH 7.2; 15 ml) for culturing at 37°C for 24 hours to confirm the presence of various wild strains of *S. mutans* ('c'—'g' types) in respect to their serological and biological characteristics by referring to, for example, biochemical and serological characteristics. After this, the live vaccine obtained according to Example 2 containing about $10^7$ living cells/ml was administered into the buccal cavity of each person 3 hours before dinner at a daily dose of about 1 ml. The administration was continued between the first and 3rd days and then effected once monthly for the following 12 months. During the test, no special treatment for preventing or inhibiting dental caries was effected except for the use of a conventional tooth paste [White & White, commercial product of Lion K.K., Tokyo, containing anhydrous silicic acid, wetting agent, bonding agent etc.] after breakfast and dinner every day. During the test period, dental plaque was optionally collected and cultured in a similar manner to that described above to investigate the adherance of the wild strains of *S. mutans* and the attenuated strain of this invention. About one to two weeks after the beginning of the administration, the wild strains of *S. mutans* ('c' to 'g' serotypes) disappeared from the dental plaque of 2 and 6 other persons, respectively. After this, no wild strain was found in the dental plaques of any of the persons treated during the test period. Moreover no ingravescence of dental caries was observed during the test. The DMF factor measured before the test was constant during the test. No significant side effects were noted following the administration of the live vaccine of this invention.

Experiment 5

Hamsters (each group consisting of 10 Golden Hamsters of 21 days old) were used to investigate the cariogenic potential of the live vaccine prepared by the method of Example 4. On each occasion, the live vaccine [0.2 ml/once every week; containing about $10^7$ living cells/ml] administered into the buccal cavity of the animal which was bred with Diet 2000 [a cariogenic diet containing sucrose, Funabashi Nojo, Japan; about 10—30 g/day]. The breeding was continued for 60, 180 or 360 days. The control groups were similarly bred without administration of the live vaccine.

Teeth samples were prepared in a similar manner to that described in Experiment 1.

The antibodies in the saliva and serum were assayed by the quantitive agglutination test using the conventional microtitre plate method. The antigen used for the agglutination test was prepared as follows. *S. mutans* Attenuated Strain K-III of this invention was inactivated with 0.2 mM glutaraldehyde and the cells were well washed by centrifugation (8000 r.p.m./20 min.) using a 1/75M phosphate buffered sodium chloride solution (pH=7.0) and suspended in a 1/75M phosphate buffered sodium chloride solution (pH=7.0) at a concentration of an optical density of 0.50 at 550 nm to obtain the antigen. The solutions were subjected to multiple dilution (×2) using the same buffer solution and then respectively divided into small fractions, (each 0.025 ml), and on each occasion, the antigen solution (each 0.025 ml) was added to the small fraction, and the mixture allowed to stand at 37°C for 4 hours in order to allow the reaction to take place. Before evaluation with the unaided eye, the mixture was left at 5°C overnight.

To determine the adherence inhibiting activity, the saliva or serum was added to a TYC medium (pH 7.2) [Stoppelaar et al: Archs. Oral Biol., 12, 1199—1201 (1967)] to give a 10-fold amount. The mixture was sterilely filtered by using a membrane filter (0.45 μ; commercial product of Millipore Corpn., U.S.A.) and subjected to multiple dilution (×2) using the same medium. On each occasion, *S. mutans* Mutant Strain K-Dp [FERM-BP 317 deposited with Bikoken (as hereinafter described) on 5th March 1982; a mutant strain having a strong cariogenicity, obtained by treating a wild strain of *S. mutans* ('c' serotype in a similar manner to that described in Example 1, followed by culturing purely] and the wild strains of *S. mutans* ('c' to 'g' serotypes) were inoculated into each of the diluted solutions for anaerobically culturing at 37°C for 48 hours by using Dott Hewuitt broth (BBL. Inc., U.S.A.; pH 7.8; 10 ml). The cultured liquor was removed from the test tube by washing with water and the cells adhered onto the tube wall were dyed with methylene blue solution to evaluate the adherence. The results are shown in Table 9.

By administering the live vaccine of this invention over an extended period of time, it was found that there was an insignificant difference between the caries scores of all molars of the test animals and those of the untreated animals, and thus it is apparent that dental caries is not substantially induced by administration of the live vaccine of this invention. Moreover no increase in the antibodies in the serum and saliva were noted nor was anything else of an unusual nature found.

TABLE 9

| Group | 1 | 2 | 3 | C1 | C2 |
|---|---|---|---|---|---|
| Test period (days) | 60 | 180 | 360 | 180 | 360 |
| Body weight of test animals (g) | 86.9 | 115 | 123 | 97.8 | 121 |
| Dental caries scores | | | | | |
| Average | 4.74 | 6.82 | 3.74 | 4.4 | 5.68 |
| 1st molars | 0 | 1.07 | 0.83 | 0 | 1.19 |
| 2nd molars | 0.99 | 2.11 | 0.84 | 1.61 | 1.72 |
| 3rd molars | 12.17 | 15.99 | 9.47 | 10.64 | 14.69 |
| Antibody titre | | | | | |
| Agglutinin A* | <4 | <4 | <4—4 | <4 | <4 |
| B** | <4 | <4 | <4 | <4 | <4 |
| Adherence activity | | | | | |
| A* | <10 | <10 | <10 | <10 | <10 |
| B** | <10 | <10 | <10—10 | <10 | <10 |

[Notes:— 2nd Control group—7 animals. *A—In the serum. **B—In the saliva].

14

Experiment 6

Gold hamsters [4 groups; each consisting of 10 animals] were used to investigate the inhibiting activity of the live vaccine of this invention on cariogenicity. The first and second groups were the test groups and the 3rd and fourth groups were the control groups. The experiment was effected for 60 days. The live vaccine used contained about $10^7$ living cells/ml.

1st group:

From the 10th to 14th days after birth, the live vaccine was continuously administered into the buccal cavity of each animal at a daily dose of 0.1 ml/once every day. From the 21st to 25th days after birth, the cultured broth of the mutant strain FERM-BP 317 having a strong cariogenic potential [obtained by culturing at 37°C for 24 hours on medium A and containing about $10^7$ living cells/ml; each 0.2 ml/once daily] was administered continuously into the buccal cavity of each animal. After the weaning period (21st day after birth), Diet 2000 [a cariogenic diet; about 10—30 g/day] was given to each animal.

2nd group:

From the 21st day after birth, the cultured broth (each 0.2 ml) of the mutant strain FERM-BP 317 containing about $10^7$ living cells/ml was administered into the buccal cavity of each animal continuously for 5 days and the animals were bred with Diet 2000 (about 10—30 g/day). On each occasion, a sample was collected from the molar surfaces of the animal and inoculated into a Mitis-salivarius medium containing bacitracin (100 µg/ml; pH about 7.2; 15 ml) for culturing at 37°C for 48 hours so as to confirm the adherence of FERM-BP 317 strains onto the teeth surfaces. From the 10th day after the completion of the administration of FERM-BP 317 strains, that is, from the 15th day after birth, the live vaccine of this invention was continuously administered into the buccal cavity at a daily dose of 0.1 ml.

Group (i):

From the 21st day to the 25th days after birth, the cultured broth of FERM-BP 317 (0.2 ml/day; containing about $10^7$ living cells/ml) was continuously administered to the buccal cavity of each animal which was bred with Diet 2000, a cariogenic diet (about 10—30 g/day).

Group (ii):

From the 21st day after birth, all animals were bred with Diet 2000 (about 10—30 g/day).

After completion of the breeding, the scores of dental caries of all molars of all animals were measured in a similar manner to that described in Experiment 5. By the quantitative agglutination reaction using the microplate method and the adherence inhibition test, the antibodies in the serum and saliva were also investigated. The results are shown in Table 10.

TABLE 10

| Group | 1 | 2 | C1 | C2 |
|---|---|---|---|---|
| Body weight in average (g) | 95 | 94.7 | 95.4 | 93.8 |
| Dental caries scores Average | 2.15 | 10.28 | 66.78 | 2.35 |
| 1st molars | 0 | 2.21 | 48.77 | 0 |
| 2nd molars | 0.21 | 7.81 | 74.48 | 0.13 |
| 3rd molars | 6.99 | 22.62 | 79.46 | 7.74 |
| Antibody titre Agglutinin A* | <4 | <4 | <4 | <4—4 |
| Agglutinin B** | <4 | <4 | <4 | <4 |
| Adherence inhibiting activity A* | <10 | <10 | <10 | <10 |
| B** | 10 | <10 | <10 | <10—10 |

[Notes:— 2nd control group—9 animals. *A—In the serum. B**—In the saliva].

15

No significant difference was found between the caries scores of all molars of Test group 1 and the Control group (ii), the cariogenic potential was inhibited by administration of the live vaccine of this invention.

Where the live vaccine was administered after settlement of FERM-BP 317 strains (Test group 2), the induction of dental caries was considerably inhibited and a significant difference was found between Test group 2 and Control group (ii). Agglutinin and adherence-inhibiting antibody were not found in the serum and saliva of the animals of any of the test groups.

## Claims

1. An inoculate for preventing or inhibiting *Streptococcus mutans* induced dental caries characterized in that said inoculate comprises as active ingredient living cells of a naturally occurring human type mutant strain of *Streptococcus mutans*, the mutant strain being effective to inhibit the growth of cariogenic wild strains of *Streptococcus mutans* and edible lactic acid-producing bacteria and being substantially deficient in cariogenic potential in the human oral cavity.

2. An inoculate as claimed in claim 1 wherein the mutant strain has a substantially reduced ability to produce dextran-like polysaccharides and is substantially deficient in cariogenic potential in a hamster's oral cavity.

3. An inoculate as claimed in claim 1 or claim 2 in association with a physiologically acceptable liquid carrier.

4. An inoculate as claimed in any one of the preceding claims wherein the living cells are obtained by purely culturing the said mutant strain.

5. An inoculate as claimed in any one of the preceding claims in the form of a food composition.

6. An inoculate as claimed in claim 5 in the form of a liquid or semi-solid food composition comprising a liquid or semi-solid physiologically acceptable carrier.

7. An inoculate as claimed in claim 6 wherein the concentration of living cells of the mutant strain in the composition is from $10^5$ to $10^8$ cells/ml of the carrier.

8. An inoculate as claimed in claim 6 or claim 7 wherein the carrier is a mammal's milk.

9. An inoculate as claimed in any of claims 5 to 8 which further contains living cells of an edible lactic acid-producing bacilli known *per se*.

10. An inoculate as claimed in claim 9 wherein the concentration of the living cells of the lactic acid-producing bacilli is from $10^7$ to $10^9$ cells/ml of the carrier.

11. An inoculate as claimed in any one of claims 5 to 10 wherein the mutant strain is *Streptococcus mutans* TMD-NC Kume (FERM-BP 319) or *Streptococcus mutans* TMD-NC 49 (FERM-BP-318).

## Patentansprüche

1. Ein Vakzin für den Schutz oder die Hemmung von durch *Streptococcus mutans* induzierter Zahnfäule, dadurch gekennzeichnet, daß das Vakzin als aktiven Bestandteil lebende Zellen eines natürlicherweise beim Menschen auftretenden mutanten Stammes von *Streptococcus mutans* aufweist, wobei der mutante Stamm wirksam zur Verhinderung des Wachstums kartogenischer wilder Stämme von *Streptococcus mutans* und eßbarer Milchsäure erzeugender Bakterien ist und im wesentlichen kein kartongenisches Potential im menschlichen Mundraum aufweist.

2. Vakzin nach Anspruch 1, bei welchem der mutante Stamm eine wesentliche verringerte Fähigkeit zur Erzeugung dextranartiger Polysaccharide und im wesentlichen kein kartogenisches Potential im Mundraum eines Hamsters aufweist.

3. Vakzin nach Anspruch 1 oder 2 zusammen mit einem physiologisch verträglichen flüssigen Träger.

4. Vakzin nach einem der voranstehenden Ansprüche, bei welchem die lebenden Zellen durch reines Kultivieren des mutanten Stammes erhalten werden.

5. Vakzin nach einem der voranstehenden Ansprüche in Form einer Lebensmittelzusammensetzung.

6. Vakzin nach Anspruch 5 in Form einer flüssigen oder halbfesten Lebensmittelzusammensetzung mit einem flüssigen oder halbfesten physiologisch verträglichen Träger.

7. Vakzin nach Anspruch 6, bei welchem die Konzentration lebender Zellen des mutanten Stamms in der Zusammensetzung zwischen $10^5$ bis $10^8$ Zellen/ml des Trägers beträgt.

8. Vakzin nach Anspruch 6 oder 7, bei welchem der Träger die Milch eines Säugetiers ist.

9. Vakzin nach einem der Ansprüche 5 bis 8, welches weiterhin lebende Zellen eines eßbaren, Milchsäure erzeugenden, an sich bekannten Bazillus enthält.

10. Vakzin nach Anspruch 9, bei welchem die Konzentration der lebenden Zellen der Milchsäure erzeugenden Bazillen $10^7$ bis $10^9$ Zellen/ml des Trägers beträgt.

11. Vakzin nach einem der Ansprüche 5 bis 10, bei welchem der mutante Stamm *Streptococcus mutans* TMD-NC Kume (FERM-BP 319) oder *Streptococcus mutans* TMD-NC 49 (FERM-BP 318) ist.

## Revendications

1. Vaccin pour la prévention ou l'inhibition de la carie dentaire induite par *Streptococcus mutans*,

16

caractérisé en ce qu'il comprend comme ingrédient actif des cellules vivantes d'une souche de mutant naturel de type humain de *Streptococcus mutans*, la souche de mutant ayant pour effect d'inhiber le développement de souches sauvages cariogéniques de *Streptococcus mutans* et de bactéries produisant de l'acide lactique, propres à la consommation, et étant notablement dépourvue de potentiel cariogénique dans la cavité buccale humaine.

2. Vaccin suivant la revendication 1, dans lequel la souche de mutant a une aptitude notablement réduite à produire des polysaccharides du type du dextrane et est notablement déficiente en potentiel cariogénique dans la cavité buccale du hamster.

3. Vaccin suivant la revendication 1 ou la revendication 2, en association avec un véhicule liquide acceptable du point de vue physiologique.

4. Vaccin suivant l'une quelconque des revendications précédentes, dans lequel les cellules vivantes sont obtenues par simple culture de ladite souche de mutant.

5. Vaccin suivant l'une quelconque des revendications précédentes, sous la forme d'une composition alimentaire.

6. Vaccin suivant la revendication 5, sous la forme d'une composition alimentaire liquide ou semi-solide comprenant un véhicule liquide ou semi-solide acceptable du point de vue physiologique.

7. Vaccin suivant la revendication 6, dans lequel la concentration des cellules vivantes de la souche de mutant dans la composition va de $10^5$ à $10^8$ cellules par millilitre de véhicule.

8. Vaccin suivant la revendication 6 ou la revendication 7, dans lequel le véhicule est le lait d'un mammifère.

9. Vaccin suivant l'une quelconque des revendications 5 à 8, qui contient en outre des cellules vivantes d'un bacille connu produisant de l'acide lactique, propre à la consommation.

10. Vaccin suivant la revendication 9, dans lequel la concentration des cellules vivantes des bacilles produisant de l'acide lactique va de $10^7$ à $10^9$ cellules/ml de véhicule.

11. Vaccin suivant l'une quelconque des revendications 5 à 10, dans leuqel la souche de mutant est *Streptococcus mutans* TMD-NC Kume (FERM-BP 319) ou *Streptococcus mutans* TMD-NC 49 (FERM-BP 318).